Europäisches Patentamt

(19) European Patent Office

Office européen des brevets

(11) Veröffentlichungsnummer: **0 005 476**
B1

# (12) EUROPÄISCHE PATENTSCHRIFT

(45) Veröffentlichungstag der Patentschrift:
30.12.81

(21) Anmeldenummer: **79101299.0**

(22) Anmeldetag: **30.04.79**

(51) Int. Cl.³: **A 61 K 45/02, C 07 G 7/00**

(54) Verfahren zur Herstellung von Humaninterferon.

(30) Priorität: **17.05.78 DE 2821466**
**17.02.79 DE 2906160**

(43) Veröffentlichungstag der Anmeldung:
**28.11.79 Patentblatt 79/24**

(45) Bekanntmachung des Hinweises auf die Patenterteilung:
**30.12.81 Patentblatt 81/52**

(84) Benannte Vertragsstaaten:
**BE CH DE FR GB IT LU NL SE**

(56) Entgegenhaltungen:
**EP-A-0 000 520**
**DE-A-2 461 379**
**US-A-3 256 152**
**US-A-3 773 924**

**NATURE, Vol. 254, 10. April 1975, Nr. 5500,**
**McMillan Journals Ltd, London, GB,**
**BENJAMIN V. SIEGEL: "Enhancement of interferon**
**production by poly(rl)poly(rC) in mouse cell cultures by**
**ascorbic acid", Seiten 531–532**

(73) Patentinhaber: **Dr. Karl Thomae GmbH, Postfach 720,**
**D-7950 Biberach (Riss) (DE)**

(72) Erfinder: **Swetly, Peter, Dr., Hietzinger**
**Hauptstrasse 40 B/9, A-1130 Wien (AT)**
Erfinder: **Adolf, Günther Rainer, Johannagasse 20/7,**
**A-1050 Wien (AT)**
Erfinder: **Bodo, Gerhard, Dr., Belghofergasse 27/5,**
**A-1120 Wien (AT)**
Erfinder: **Lindner-Frimmel, Silvia Jutta, Dr.,**
**Soosenstrasse 6, A-2380 Perchtoldsdorf (AT)**
Erfinder: **Meindl, Peter, Dr., Hockegasse 63/1,**
**A-1180 Wien (AT)**
Erfinder: **Tuppy, Hans, Prof. Dr., Amalgergasse 10,**
**A-1190 Wien (AT)**

## Verfahren zur Herstellung von Humaninterferon

Die Herstellung von Humaninterferon aus Zellen humanen Ursprungs hat aufgrund der medizinischen Bedeutung der Interferone bei der Behandlung akuter und chronischer Virusinfektionen sowie bei der Krebstherapie an Menschen weltweite Beachtung gefunden. Die hierfür benötigten Mengen von Interferonen können jedoch durch die bisher bekannten Verfahren zu ihrer Herstellung nicht zur Verfügung gestellt werden, da bei diesen nach Induktion der Gewebekulturzellen nur eine niedrige Konzentration von Humaninterferonen erreicht werden kann.

Die Herstellung der Interferone erfolgt bei den bisher bekannten Verfahren durch Inkubation mit einem Interferoninduktor, z. B. einem viralen Induktor oder einem Nukleinsäureinduktor, behandelter Zellen in einem Zellkulturmedium. Anschliessend werden die Zellen abgetrennt und gegebenenfalls die verwendeten Induktor-Viren durch Zugabe einer Mineralsäure und Stehen während mehrerer Tage zerstört. Die so gewonnene Lösung von Rohinterferon wird anschliessend mittels herkömmlicher Reinigungsschritte konzentriert und gereinigt (siehe beispielsweise DE-A-2 724 918).

Als Zellen für die Interferoninduktion werden hierbei üblicherweise entweder eine permanent kultivierbare Zellinie lymphoblastoiden Ursprungs, z. B. Namalwa-Zellen (siehe G. Klein et al. in Intern. J. Cancer 10, 44–57 [1972]), oder menschliche Fibroblastenzellen entweder mit diploidem Chromosomensatz und beschränkter Lebensdauer unter Zellkulturbedingungen oder mit heteroploidem Chromosomensatz und permanenter Teilbarkeit verwendet.

Als Induktor-Viren werden üblicherweise Paramyxoviren wie haemagglutinierendes Virus Japan (HVJ-Sendai Virus) oder Newcastle disease Virus, Rhabdoviren wie vesiculäres Stomatitis-Virus, Masernviren oder Viren der Reovirusgruppe wie Reovirus oder Blue tongue Virus, eingesetzt, als Nukleinsäureinduktoren, z. B. eine doppelsträngige Ribonukleinsäure wie poly I:C, gegebenenfalls in Kombination mit metabolischen Inhibitoren und/oder Polykationen, verwendet.

Des weiteren wird in der Literatur beschrieben, dass die Zugabe von L-Glutamin (siehe DE-A-2 461 379) oder von Ascorbinsäure (siehe Nature 254 [1975] S. 531) bei der Interferonproduktion zu einer Steigerung der Ausbeute an Interferon führen würde. Dies konnte für Humanzellen lymphoblastoiden Ursprungs nicht bestätigt werden (siehe Beispiel 1).

Überraschenderweise wurde nun gefunden, dass die Zugabe einer sogenannten Stimulatorsubstanz oder eines Gemisches derartiger Substanzen zu geeigneten Zellen zu einer Steigerung der produzierten Interferonmenge auf ein Vielfaches, z. B. auf das 4- bis 60-fache, der Ausbeute ohne Vorbehandlung führt. Die Zugabe des Stimulators erfolgt hierbei vorzugsweise vor der Interferonherstellung mit Interferoninduktoren.

Hierbei ist es besonders überraschend, dass die erfindungsgemässe Ausbeutesteigerung an Interferon sowohl auf einer Erhöhung des Prozentsatzes an Interferon-produzierenden Zellen als auch auf einer Steigerung der Interferonausbeute pro Zelle beruht.

Als Stimulatoren haben sich hierbei insbesondere solche Substanzen bewährt, durch deren Zusatz zum Zellkulturmedium der Prozentsatz derjenigen Zellen, welche Interferon produzieren, erhöht wird. Hierunter sind solche Substanzen zu verstehen, deren Zusatz zum Zellkulturmedium bewirkt, dass Zellen, welche sich in logarithmischer Wachstumsphase befinden, bei optimalen Wachstumsbedingungen aus der Wachstumsphase in eine Ruhephase übergehen, welche dadurch definiert ist, dass die Zellen bezüglich ihres Desoxyribonukleinsäure (DNA)-Gehalts postmitotischen (G1-Phase) Zellen entsprechen und keine DNA-Synthese abläuft. Dieser postmitotische Zustand lässt sich dabei durch cytophotometrische Bestimmung des DNA-Gehalts einzelner Zellen nach einer beliebigen literaturbekannten Methode charakterisieren (siehe beispielsweise Cytobiologie 15, 357–362 [1977]). Der Prozentsatz DNA-synthetisierender Zellen wird am einfachsten durch Einbau von $^3$H-Thymidin in Trichloressigsäure fällbares zellulares Material bestimmt. So zeigen beispielsweise Zellen, die bei einer Zelldichte um $1 \times 10^6$ Zellen/ml kultiviert werden, eine maximale DNA-Synthese.

Als Stimulatorsubstanzen haben sich insbesondere gesättigte, aliphatische Carbonsäuren, z. B. Alkansäuren mit 3-6 Kohlenstoffatomen, wie Propionsäure, n-Buttersäure, Isobuttersäure, n-Valeriansäure, Capronsäure und deren Salze mit anorganischen oder organischen Basen, N,N'-diacetylierte Diamine mit 4 bis 8 Kohlenstoffatomen, insbesondere jedoch deren $\alpha,\omega$-Derivate wie beispielsweise Pentamethylenbisacetamid, Hexamethylenbisacetamid oder Heptamethylenbisacetamid, organische Schwefelverbindungen, z. B. deren Sulfoxide wie Dimethylsulfoxid, oder Glucocorticoide, welche in 11β-Stellung durch eine hydrophile Gruppe wie eine Hydroxy- oder Hydroxymethylgruppe substituiert sind, z. B. Hydrocortison, Dexamethason, Prednisolon, Aldosteron, Triamcinolon, 11-Des - oxycortison oder Prednison,als geeignet erwiesen. Die Endkonzentration dieser Stimulatoren beträgt zweckmässigerweise 0,01 µMol–300 mMol/l. Die bevorzugte Endkonzentration bei einer als Stimulator verwendeten Alkansäure beträgt jedoch 0,5–5 mMol/l, bei einem diacetylierten Diamin 1-10 mMol/l bei einer organischen Schwefelverbindung wie Dimethylsulfoxid 50-300 mMol/l und bei Glucocorticoiden 0,01 µMol–10 µMol/l.

Als besonders geeignete Stimulatoren haben sich hierbei Propionsäure, n-Buttersäure, n-Valeriansäure und deren Alkalisalze wie die Natriumsalze, Hexamethylenbisacetamid, Dimethylsulfoxid, Dexamethason, Triamcinolon, Hydrocortison und Prednisolon erwiesen.

In den benannten Vertragsstaaten Belgien, Schweiz, Bundesrepublik Deutschland, Frankreich, Grossbritannien, Niederlande und Schweden gehört gem. Art. 54 (3) EPÜ auch die EP-A 0 000 520 zum Stande der Technik, die die Verwendung von Alkancarbonsäuren, insbesondere Buttersäure, in einem entsprechenden Verfahren zur Herstellung von Humaninterferon beschreibt. Für diese Staaten gelten daher besondere Patentansprüche.

Das erfindungsgemässe Verfahren wird wie folgt besonders bevorzugt durchgeführt:

Namalwa-Zellen werden in einem Wachstummedium, vorzugsweise dem Medium RPMI 1640 (Firma Gibco, USA, oder Firma Flow, Grossbritannien, dieses enthält 300 mg/l L-Glutamin) vermehrt, welches als Zusätze 10–25 Vol. %, vorzugsweise jedoch 2–6 Vol. %, vorgereinigtes Humanserum enthält. Hierbei kann alternativ zu Humanserum auch ein Zusatz von Serum oder Serumfraktionen anderer Species verwendet werden, z. B. foetales Rindserum in einer Konzentration von 5–15 Vol. %.

Die derart kultivierten Zellen werden in Suspension bei einer Dichte von 0,2–5 × $10^6$ Zellen/ml, vorzugsweise aber bei einer Dichte von 0,5–1 × $10^6$ Zellen/ml, mit einer Stimulatorverbindung, z. B. einem Alkalisalz der n-Buttersäure, in einer Endkonzentration von zweckmässigerweise 1 mMol/l, versetzt und zweckmässigerweise bei einer Temperatur von 35–37 °C inkubiert. Nach einem Zeitraum von 6–72 Stunden, vorzugsweise jedoch von 24–48 Stunden, werden die Zellen durch Zentrifugation gewonnen und zweckmässigerweise bei einer Dichte von 50 × $10^6$ Zellen/ml Medium, ohne Stimulatorsubstanz, suspendiert. Diese Suspension wird beispielsweise mit HVJ-Virus als Interferoninduktor infiziert und inkubiert. Nach ungefähr 1–3 Stunden werden die infizierten Zellen durch Zentrifugation gesammelt und bei einer Dichte von 5–10 × $10^6$ Zellen/ml in einem serumfreien Zellkulturmedium, z. B. in «Minimum Essential Medium-Eagle»- Zellkulturmedium (Firma Gibco, USA, oder Firma Flow, Grossbritannien), für einen Zeitraum von 15–24 Stunden, vorzugsweise aber 18–20 Stunden, in Abwesenheit der Stimulatorsubstanz bei 35–39 °C und einem pH-Wert des Gewebekulturmediums von 6,7–8,0, vorzugsweise jedoch von 7,3, inkubiert. Die erhaltene Lösung des lymphoblastoiden Rohinterferons wird durch Zentrifugation von den Namalwa-Zellen abgetrennt und zur Inaktivierung restlicher Induktor-Viren durch Zugabe von Mineralsäure, beispielsweise von Salzsäure auf ein pH-Wert von vorzugsweise 2,0 eingestellt und mehrere Tage, z. B. 3–5 Tage, bei 0–4 °C inkubiert.

Zur weiteren Reinigung kann die so gewonnene Lösung von lymphoblastoiden Rohinterferon beispielsweise folgenden Reinigungsschritten unterzogen werden:

a) Die erhaltene Lösung wird beispielsweise mit Natronlauge neutralisiert und das Interferon mit einem Zinksalz, z. B. Zinkacetat, gefällt und mittels Zentrifugation abgetrennt. Das erhaltene Präzipitat wird in kalter verdünnter Salzsäure, z. B. 0,1 N Salzsäure, in Lösung gebracht.

b) Nach Klärung der salzsauren Lösung mittels erneuter Zentrifugation wird diese mit einem Ionenaustauscher, vorzugsweise Ionenaustauscher SP-Sephadex C-25 (eingetragenes Warenzeichen der Firma Pharmacia Fine Chemicals [Uppsala]) versetzt und mehrere Stunden gerührt. Nachdem das Interferon vom Ionenaustauscher aufgenommen wurde, wird dieser mehrmals zur vollständigen Entfernung der Zinkionen mit einem Puffer gewaschen, z. B. 3-mal mit 0,1 Mol/l Glycin/Salzsäure-Puffer pH 2,5 und dreimal mit 0,1 Mol/l Kaliumzitrat pH 4,0, dem 0,005 Mol/l Dinatriumäthylendiamintetraacetat zugesetzt war. Anschliessend wird der Ionenaustauscher mit dem Zitratpuffer in eine Chromatographiesäule gefüllt und das Interferon mit einem schwach basischen Puffer, z. B. mit 0,1 Mol/l Kalium-phosphat pH 8, dem 1 Mol/l Kaliumchlorid zugesetzt war, eluiert.

c) Die gesammelten proteinhaltigen Eluate werden mit Salzsäure bei niederen Temperaturen zweckmässigerweise auf pH 3,5 eingestellt und nach Entfernung der entstandenen Trübung mittels Zentrifugation wird zur Fällung des Interferons eine Lösung von Kaliumthiocyanat zugesetzt. Der gebildete Niederschlag wird absitzen gelassen, durch Zentrifugieren gesammelt und in einem möglichst kleinen Volumen eines schwach basischen Puffers, z. B. 0,1 Mol/l Kaliumphosphat pH 8, aufgenommen und die Lösung ultrazentrifugiert.

d) Die so gewonnene Lösung wird über eine Chromatographiesäule weiter gereinigt. Hierzu wird zweckmässigerweise ein Molekularsieb wie Sephadex G-100 (eingetragenes Warenzeichen der Firma Pharmacia Fine Chemicals [Uppsala]) mit der Teilchengrösse 40–120 μ und als Eluens Phosphat-gepufferte physiologische Kochsalzlösung, der 0,001 Mol/l Dinatrium-äthylendiamintetraacetat zugegeben ist, verwendet. Hierbei ist es zweckmässig, vor Verwendung der Säule diese mit Proteinen von bekanntem Molgewicht zu eichen. Es werden die Fraktionen des Eluates gesammelt, die einem Molekulargewicht von 10 000–35 000 Daltons entsprechen.

e) Die gesammelten Fraktionen werden mittels Salzsäure vorzugsweise auf pH 3 eingestellt und über eine kleine Säule, gefüllt mit einem Ionenaustauscher wie SP-Sephadex C-25 (eingetragenes Warenzeichen der Firma Pharmacia Fine Chemicals [Uppsala]), geleitet, welche zweckmässigerweise mit 0,1 Mol/l Kaliumzitrat pH 3,0 vorgewaschen wird. Bei diesem pH wird das Interferon von der Säule festgehalten und anschliessend nach kurzem Waschen mit dem Zitrat-Puffer mit einem Phosphat-Puffer, z. B. 0,1 Mol/l Kaliumphosphat pH 8, eluiert. Die proteinhaltigen Fraktionen werden gesammelt und gegen Phosphat-gepufferte physiologische Kochsalzlösung dialysiert.

Die nachfolgenden Beispiele sollen die Erfindung näher erläutern, jedoch nicht den Umfang der Erfindung einschränken:

Vorbemerkung:

Die «Namalwa-Zellen» werden in Suspensionskultur in einem Fermentor vermehrt. Hierbei wird als Wachstumsmedium das Medium RPMI 1640, dem 20 Vol. % Tryptose-Phosphat-Broth und 2-4 Vol. % vorgereinigtes Humanserum beigegeben sind, verwendet. Das vorgereinigte Humanserum wird durch Fällung eines Teiles der Serumproteine mit 6% Polyäthylenglykol 6000 hergestellt (siehe Inglot et al. in Acta Virol 19, 250-254 [1975]). Die erreichten Zelldichten liegen bei $2-7 \times 10^6$ Zellen/ml.

Das «Sendai-Virus» wird in embryonierten Hühnereiern vermehrt, die Reinigung erfolgt mittels einer High-Speed Zentrifuge und die Suspension im Wachstumsmedium mittels Ultraschall Behandlung. Das «Sendai-Virus» wird bei $-70\,°C$ aufbewahrt.

Beispiel 1

Namalwa-Zellen, in exponentiellem Wachstumsstadium wurden in 85 l Wachstumsmedium bei einer Dichte von $5 \times 10^5$ Zellen/ml suspendiert. Die Stimulatorsubstanz wurde für einen Zeitraum von t Stunden bei $37\,°C$ in einer Konzentration von c mMol/l zum Zellkulturmedium zugesetzt. Während dieser Vorbehandlungszeit geht die Rate der DNA-Synthese bezogen auf die DNA-Synthese in unbehandelten Zellen auf unter 2% zurück und die Zellen sind in einem bezüglich der DNA-Synthese definierten, Ruhezustand, welcher der G-1 Phase des Zellzyklus entspricht. Nach der Vorbehandlungszeit wurden die Zellen durch Zentrifugation ($700 \times g$, 15 Minuten) gesammelt und bei einer Dichte von $5 \times 10^7$ Zellen/ml in 1,7 Litern Wachstumsmedium bei $37\,°C$ 2 Stunden in Rollkulturflaschen mit $2^{10}$ Haemagglutinationseinheiten HVJ/ml inkubiert. Die auf diese Weise induzierten Zellen wurden durch Zentrifugation gesammelt und bei einer Dichte von $5 \times 10^6$ Zellen/ml in 17 Litern serumfreiem Eagle's-Minimum Essential Medium (mit Earle Salzlösung) bei $35,5\,°C$ 20 Stunden lang bei pH 7.3 inkubiert. Hierzu wurden 20 l Rührkolben eingesetzt. Die Rohlösung (=Zellüberstand) wurde nach Abzentrifugieren der Zellen ($2000 \times g$, 30 Minuten) bei einer Temperatur von maximal $10\,°C$ mit 5N Salzsäure auf pH 2 eingestellt und 3-5 Tage bei $4\,°C$ zur Inaktivierung des verwendeten HVJ gelagert.

Die nachfolgende Tabelle enthält die gefundene Ausbeutesteigerung an Interferon bei Zugabe von c mMol/l n-Buttersäure, Ascorbinsäure oder L-Glutamin als Stimulator:

| Konzentration c mMol/l | | Dauer der Vorbehandlung t Stunden | IE/$10^6$Z+ | Faktor |
|---|---|---|---|---|
| 0++ | n-Butter- | | 280 | 1 |
| 1 | säure | 26 | 7 270 | 26 |
| 0++ | n-Butter- | | 1 480 | 1 |
| 2 | säure | 40 | 55 550 | 38 |
| 0++ | n-Butter- | | 1 100 | 1 |
| 1 | säure | 48 | 36 000 | 33 |
| 2,5 | | 48 | 65 520 | 60 |
| 0++ | n-Butter- | | 1 630 | 1 |
| 1 | säure | 68 | 44 000 | 27 |
| 1 | Ascorbin- | 24 | zelltoxisch | — |
| 0,1 | säure | 24 | 500 | 1 |
| 0,01 | | 24 | 650 | 1 |
| 10 | L-Glutamin | 24-72 | 800 | 1 |
| 20 | | 24-72 | 275 | $< 1$ |

+Internationale Einheiten Interferon pro $10^6$ Zellen.
++ enthält 300 mg/l L-Glutamin ($\triangleq$ ca. 2 mMol/l)

Beispiel 2

Namalwa-Zellen wurden als Suspensionskultur in 85 l Wachstumsmedium bis zum Erreichen einer Zelldichte von $5 \times 10^5$ Zellen/ml kultiviert. In diesem Stadium befinden sich die Zellen in der exponentiellen Wachstumsphase, welche sich durch ein Maximum an DNA-Synthese pro Zelle auszeichnet. Die Stimulatorsubstanz wurde nun als wässrige Lösung für einen Zeitraum von t Stunden bei $35,5-37\,°C$ in einer Konzentration von c mMol/l zum Zellkulturmedium zugesetzt. Nach dieser Vorbehandlungszeit, innerhalb derer die DNA-Syntheserate auf weniger als 12% einer nicht mit Stimulatorsubstanz behandelten Kontrollkultur gesunken war und der Grossteil der Zellen in einer der G1-Phase des Zellzyklus entsprechenden Ruhephase angesammelt war, wurden die Zellen durch Zentrifugation bei $700 \times g$ in einem kontinuierlichen Durchflusszentrifugationsverfahren sedimentiert. Die derart gesammelten Zellen wurden bei einer Dichte von $1 \times 10^7$ Zellen/ml in 9 l Wachstumsmedium, welches c

mMol/l Stimulatorsubstanz enthielt, suspendiert und 4 Stunden lang bei 35,5–37 °C in einem Rührkolben mit $2^8$ Hämagglutinationseinheiten HVJ/ml inkubiert. Die auf diese Weise induzierten Zellen wurden durch Zentrifugation gesammelt und bei einer Dichte von $5 \times 10^6$ Zellen/ml in 18 l serumfreien Eagle's Minimum Essential Medium (mit Earle Salzen) suspendiert und bei 36 °C 24 Stunden lang bei pH 7.1 in einem 20 l Rührkolben inkubiert. Nach Abzentrifugieren der Zellen ($2000 \times g$, 30 min) stellt der Zellüberstand die Interferonrohlösung dar. Diese wurde mit 5 N Salzsäure auf pH 2 eingestellt und 3–5 Tage bei 4 °C zur Inaktivierung des viralen Induktors gelagert.

Die nachfolgende Tabelle enthält die gefundene Ausbeutesteigerung an Interferon bei Zugabe von c mMol/l Hexamethylenbisacetamid als Stimulator:

| Konzentration c mMol/l | Dauer der Vorbehandlung t Stunden | IE/$10^6$Z[+] | Faktor |
|---|---|---|---|
| 0[++] | | 2 400 | 1 |
| 2,5 | 72 | 10 400 | 4 |
| 0[++] | | 1 480 | 1 |
| 5 | 40 | 29 800 | 20 |

[+] Internationale Einheiten Interferon pro $10^6$ Zellen.
[++] enthält 300 mg/l L-Glutamin ($\triangleq$ ca. 2 mMol/l)

### Beispiel 3

Namalwa-Zellen wurden als Suspensionskultur in 85 l Wachstumsmedium bis zum Erreichen einer Zelldichte von $1 \times 10^6$ Zellen/ml kultiviert. Die Zellen befinden sich in der exponentiellen Wachstumsphase, welche sich durch optimale DNA-Syntheserate auszeichnet. Stimulatorsubstanzen wurden in Form einer neutralen Lösung für einen Zeitraum von 48 Stunden bei 35,5–37 °C in folgender Mischung zum Wachstumsmedium zugegeben: Propionsäure 2,5 mMol/l, Hydrocortison 1 µMol/l. Die Syntheserate war innerhalb der 48-stündigen Behandlungszeit auf weniger als 5% einer nicht mit dem Stimulatorsubstanzengemisch behandelten Kontrollkultur gesunken und der Grossteil der Zellen in einer der G1-Phase des Zellzyklus entsprechenden Ruhephase angesammelt. Nach der Vorbehandlungszeit wurden die Zellen bei $700 \times g$ in einem kontinuierlichen Durchflusszentrifugationsverfahren sedimentiert. Die derart gesammelten Zellen wurden bei einer Dichte von $4,5 \times 10^7$ Zellen/ml in 2 l Wachstumsmedium suspendiert und 2 Stunden lang mit $2^{10}$ Haemagglutinationseinheiten HVJ/ml in Rollkulturflaschen bei 37 °C inkubiert. Nach dieser Zeit wurden die Zellen gesammelt und bei einer Dichte von $1 \times 10^6$ Zellen/ml in 85 l serumfreien Wachstumsmedium suspendiert und 36 Stunden bei pH 7.3 in Suspensionskultur bei 35,5–37 °C gehalten. Nach Abtrennen der Zellen bei $2000 \times g$ in einem kontinuierlichen Durchflusszentrifugationsverfahren wurde der Zellüberstand gesammelt, mit 5N Salzsäure bei höchstens 10 °C auf pH 2 eingestellt und 3–5 Tage bei 4 °C gelagert. Die so gewonnene Rohinterferonlösung zeichnete sich durch einen um einen Faktor 22 gegenüber einer nicht vorbehandelten Kontrollkultur aus (Kontrollkultur: 1050 IE Interferon/$10^6$ Zellen; mit Propionsäure und Hydrocortison behandelte Kultur: 23200 IE Interferon/$10^6$ Zellen.

### Beispiel 4

Analog den vorstehenden Beispielen wurde bei einer Zelldichte von $5 \times 10^5$ Namalwazellen/ml und einer Vorbehandlungszeit von 48 Stunden mit folgenden Stimulatorsubstanzen eine Ausbeutesteigerung um den Faktor X gegenüber Kontrollkulturen erzielt:

| Stimulator | Konzentration mMol/l | Faktor (X) |
|---|---|---|
| Propionsäure | 1 | 4 |
| n-Buttersäure | 1 | 29 |
| n-Valeriansäure | 1 | 4 |
| Propionsäure | 2,5 | 8 |
| Propionsäure | 5 | 31 |
| n-Buttersäure | 5 | 29 |
| n-Valeriansäure | 5 | 22 |
| Capronsäure | 5 | 8 |
| Dimethylsulfoxid | 140 | 4 |
| Dimethylsulfoxid | 280 | 16 |

### Beispiel 5

In Analogie zu den vorhergehenden Beispielen wurden Namalwa-Zellen bei einer Dichte von $5 \times 10^5$ Zellen/ml mit den folgenden Stimulatorsubstanzen behandelt, wodurch eine Steigerung der Interferonausbeute gegenüber unbehandelten Kulturen um den Faktor X erhalten wurden:

| Stimulator | Konzentration µMol/l | Faktor X |
|---|---|---|
| Prednisolon | 10 | 36 |
| | 1 | 40 |
| | 0,1 | 6 |
| Dexamethason | 10 | 44 |
| | 1 | 29 |

| Stimulator | Konzentration μMol/l | Faktor X |
|---|---|---|
| | 0,1 | 31 |
| | 0,01 | 14 |
| Triamcinolon | 10 | 19 |
| | 1 | 16 |
| | 0,1 | 12 |
| Hydrocortison | 10 | 50 |
| | 1 | 17 |
| | 0,1 | 4 |
| 11-Desoxycortison | 10 | 4 |
| Prednison | 10 | 3,5 |

Das gemäss den Beispielen 1–5 hergestellte Rohinterferon kann beispielsweise wie folgt abgetrennt und/oder gereinigt werden:

a) Konzentration mit Zinkacetat 17,2 Liter Rohinterferon werden mit 2N Natronlauge neutralisiert, 1 Mol/l Zinkacetat bis zu einer Konzentration von 0,02 Mol/l unter Rühren zugegeben, und das pH mit Natronlauge auf 7,2 eingestellt. Das entstandene Präzipitat enthält das Interferon. Es wird 2 Stunden absitzen gelassen und der Überstand so weit als möglich abgesaugt. Der Niederschlag wird sodann durch Zentrifugieren gewonnen und mit 640 ml eisgekühlter 0,1 N Salzsäure bis zur Lösung digeriert, wobei das pH auf 2,5 eingestellt wird. Die trübe Lösung wird in der Zentrifuge geklärt (bei 2000 g, 60 Minuten).

b) 1. Chromatographie am Ionenaustauscher

Zu 653 ml der sauren Lösung des Interferons werden 25,8 g Ionenaustauscher SP-Sephadex C-25 (eingetragenes Warenzeichen der Firma Pharmacia Fine Chemicals [Uppsala]), zugegeben und die Mischung über Nacht im Kühlraum gerührt. Das Interferon wird dabei vom Ionenaustauscher aufgenommen. Dieser wird sodann 3-mal mit jeweils 1 Liter 0,1 Mol/l Glycin/Salzsäure-Puffer pH 2,5 und anschliessend 3-mal mit jeweils 1 Liter 0,1 Mol/l Kalium-zitrat pH 4,0 (dem 0,005 Mol/l Dinatriumäthylendiamin-tetraacetat zugesetzt sind) dekantiert, bis keine Zinkionen mehr mit Dithizon im Waschwasser nachweisbar sind. Der Ionenaustauscher wird sodann mit dem Zitratpuffer in eine Chromatographiesäule gefüllt und das Interferon mit 0,1 Mol/l Kalium-phosphat pH 8,0, welches 1 Mol/l Kaliumchlorid enthält, eluiert. Die proteinhaltigen Fraktionen werden gesammelt (210 ml), wobei als Indikator die Extinktion bei 280 nm ($E_{280\,nm}$) verwendet wird.

c) Konzentrierung mit Kalium-thiocyanat Die gesammelten Fraktionen (210 ml) werden mit Salzsäure bei 4°C auf pH 3,5 eingestellt und die entstandene Trübung durch Zentrifugieren entfernt. Zu der Lösung werden hierauf 1/10 des Volumens an 10 Mol/l Kalium-thiocyanat zugesetzt. Der entstehende Niederschlag, der das Interferon enthält, wird 30 Minuten absitzen gelassen, durch Zentrifugieren gesammelt, und in 3 ml 0,1 Mol/l Kalium-phosphat pH 8 aufgenommen. Die Lösung wird durch Ultrazentrifugieren geklärt (bei 70000 g, 80 Minuten).

d) Gelfiltration Eine Chromatographiesäule von 5 × 50 cm wird mit Sephadex G-100 (eingetragenes Warenzeichen der Firma Pharmacia Fine Chemicals [Uppsala]), Teilchengrösse 40–120 μ, gefüllt, welches mit Phosphat-gepufferter physiologischer Kochsalzlösung (PBS) unter Zusatz von 0,001 Mol/l Dinatrium-äthylendiamintetraacetat gequollen wurde. Die Chromatogrpahie erfolgt im Kühlraum mit Phosphat gepufferter physiologischer Kochsalzlösung/Äthylen-diamintetraacetat als Eluiermittel und 40 ml/Stunde Flussgeschwindigkeit. Jede neugefüllte Säule wird vor Verwendung mit Proteinen von bekanntem Molgewicht geeicht. Das Interferon-Konzentrat (3,5 ml) wird unter denselben Bedingungen nach der Molekülgrösse der Komponenten fraktioniert und die Fraktionen des Eluates, die einem Molekulargewicht von 10000–35000 Daltons entsprechen, gesammelt (30,5 ml). In diesem Bereich ist die gesamte Interferon-Aktivität enthalten (Molgewicht etwa 22000).

e) 2. Chromatographie am Ionenaustauscher

Die gesammelten Fraktionen (30,5 ml) werden mit Salzsäure auf pH 3 eingestellt und über eine kleine (1 × 7 cm) Säule von SP-Sephadex C-25 (eingetragenes Warenzeichen der Firma Pharmacia Fine Chemicals [Uppsala]) gepumpt, welche mit 0,1 Mol/l Kalium-zitrat pH 3,0 vorgewaschen wurde. Das Interferon wird bei diesem pH von der Säule festgehalten. Nach kurzem Waschen mit dem Zitratpuffer wird das Interferon mit 0,1 Mol/l Kalium-phosphat pH 8 eluiert. Die proteinhaltigen Fraktionen werden nach der $E_{280\,nm}$ des Eluates gesammelt, gegen Phosphat-gepufferte physiologische Kochsalzlösung dialysiert und bei –20°C aufgehoben. Das Volumen des gereinigten Konzentrats beträgt 3,9 ml.

**Patentansprüche für die Vertragsstaaten:** BE, DE, FR, GB, NL, SE, CH

1. Verfahren zur Herstellung von Interferon, dadurch gekennzeichnet, dass Zellen einer permanent kultivierbaren Zellinie lymphoblastoiden Ursprungs, bevor diese mit viralen Interferoninduktoren inkubiert werden, mit Stimulatoren behandelt werden, die bewirken, dass die lymphoblastoiden Zellen, welche sich in logarithmischer Wachstumsphase befinden, bei optimalen Wachstumsbedingungen aus der Wachstumsphase in eine Ruhephase übergehen, welche dadurch definiert ist, dass die Zellen bezüglich ihres Desoxyribonukleinsäuregehalts postmitotischen ($G_1$-Phase) Zellen entsprechen und keine DNA-Synthese abläuft, und gewünschtenfalls das bei der Inkubation gebildete Interferon abgetrennt und/oder nach an sich bekannten Methoden gereinigt wird, wobei die Verwendung von geradkettigen gesättigten Stimulatoren ausgenommen ist.

2. Verfahren gemäss Anspruch 1, dadurch gekennzeichnet, dass als Stimulator diacetylierte Diamine, organische Sulfoxide oder ein Glucocorticoid, welches in 11β-Stellung durch eine hydrophile Gruppe substituiert ist, sowie deren Gemische oder ein Gemisch bestehend aus den vorstehend erwähnten Stimulatoren und einer gesättigten aliphatischen Carbonsäure und deren Salzen verwendet werden.

3. Verfahren gemäss Anspruch 1, dadurch gekennzeichnet, dass als Stimulator, N,N'-diacetylierte α,ω-Diamine mit 4 bis 8 Kohlenstoffatomen, Dimethylsulfoxid oder Glucocorticoide, welche in 11β-Stellung durch eine Hydroxy- oder Hydroxymethylgruppe substituiert sind, sowie deren Gemische oder ein Gemisch bestehend aus den vorstehend erwähnten Stimulatoren und einer Alkansäure mit 3 bis 6 Kohlenstoffatomen und deren Salzen verwendet werden.

4. Verfahren gemäss Anspruch 1, dadurch gekennzeichnet, dass als Stimulator Pentamethylenbisacetamid, Hexamethylenbisacetamid, Heptamethylenbisacetamid, Dimethylsulfoxid, Prednisolon, Dexamethason, Triamcinolon, Hydrocortison, 11-Desoxycortison und Prednison, sowie deren Gemische oder ein Gemisch bestehend aus den vorstehend erwähnten Stimulatoren und Propionsäure, n-Buttersäure, Isobuttersäure, n-Valeriansäure, Capronsäure und deren Salzen mit anorganischen oder organischen Basen verwendet werden und der Stimulator zu den Zellen für einen Zeitraum von 6–72 Stunden, vorzugsweise jedoch 24–48 Stunden, vor der Zugabe der Interferoninduktoren zugegeben wird.

5. Verfahren gemäss den Ansprüchen 1–4, dadurch gekennzeichnet, dass die Endkonzentration des Stimulators 0,01 µMol–300 mMol/l beträgt.

6. Verfahren gemäss den Ansprüchen 1–4, dadurch gekennzeichnet, dass als Stimulator N,N'-diacetylierte Diamine mit 4 bis 8 Kohlenstoffatomen und/oder Dimethylsulfoxid in einer Endkonzentration von 0,1 µMol/l bis 250 mMol/l verwendet werden.

**Patentansprüche für die Vertragsstaaten: IT, LU**

1. Verfahren zur Herstellung von Interferon, dadurch gekennzeichnet, dass Zellen einer permanent kultivierbaren Zellinie lymphoblastoiden Ursprungs, bevor diese mit viralen Interferoninduktoren inkubiert werden, mit Stimulatoren behandelt werden, die bewirken, dass die lymphoblastoiden Zellen, welche sich in logarithmischer Wachstumsphase befinden, bei optimalen Wachstumsbedingungen aus der Wachstumsphase in eine Ruhephase übergehen, welche dadurch definiert ist, dass die Zellen bezüglich ihres Desoxyribonukleinsäuregehalts postmitotischen (G$_1$-Phase) Zellen entsprechen und keine DNA-Synthese abläuft, und gewünschtenfalls das bei der Inkubation gebildete Interferon abgetrennt und/oder nach an sich bekannten Methoden gereinigt wird.

2. Verfahren gemäss Anspruch 1, dadurch gekennzeichnet, dass als Stimulator eine gesättigte aliphatische Carbonsäure und deren Salze, diacetylierte Diamine, organische Sulfoxide oder ein Glucocorticoid, welches in 11β-Stellung durch eine hydrophile Gruppe substituiert ist, sowie deren Gemische verwendet werden.

3. Verfahren gemäss Anspruch 1, dadurch gekennzeichnet, dass als Stimulator eine Alkansäure mit 3 bis 6 Kohlenstoffatomen oder deren Salze, N,N'-diacetylierte α,ω-Diamine mit 4 bis 8 Kohlenstoffatomen, Dimethylsulfoxid oder Glucocorticoide, welche in 11β-Stellung durch eine Hydroxy- oder Hydroxymethylgruppe substituiert sind, sowie deren Gemische verwendet werden.

4. Verfahren gemäss Anspruch 1, dadurch gekennzeichnet, dass als Stimulator Propionsäure, n-Buttersäure, Isobuttersäure, n-Valeriansäure, Capronsäure und deren Salze mit anorganischen oder organischen Basen, Pentamethylenbisacetamid, Hexamethylenbisacetamid, Heptamethylenbisacetamid, Dimethylsulfoxid, Prednisolon, Dexamethason, Triamcinolon, Hydrocortison, 11-Desoxycortison und Prednison, sowie deren Gemische verwendet werden und der Stimulator zu den Zellen für einen Zeitraum von 6–72 Stunden, vorzugsweise jedoch 24–48 Stunden, vor der Zugabe der Interferoninduktoren zugegeben wird.

5. Verfahren gemäss den Ansprüchen 1–4, dadurch gekennzeichnet, dass die Endkonzentration des Stimulators 0,01 µMol–300 mMol/l beträgt.

6. Verfahren gemäss den Ansprüchen 1–4, dadurch gekennzeichnet, dass als Stimulator eine Alkansäure mit 3 bis 5 Kohlenstoffatomen oder deren Salze, N,N'-diacetylierte Diamine mit 4 bis 8 Kohlenstoffatomen und/oder Dimethylsulfoxid in einer Endkonzentration von 0,1 µMol/l bis 250 mMol/l verwendet werden.

**Claims for the Contracting States: BE, DE, FR, GB, NL, SE, CH**

1. Process for the preparation of interferon, characterised in that, before being incubated with viral interferon inducers cells of a permanently cultivatable line of cells of lymphoblastoid origin are treated with stimulators which cause the lymphoblastoid cells which are in a logarithmic growth phase to move out of the growth phase into a resting stage, under optimum growth conditions, this resting stage being defined by the fact that the cells correspond to post-mitotic (G$_1$ phase) cells with regard to their deoxyribonucleic acid content and no DNA synthesis occurs, and if desired the interferon formed during the incubation is separated and/or purified by methods known per se, with the proviso that straight-chained saturated aliphatic carboxylic acids or the salts thereof are not used as sole stimulators.

2. Process as claimed in claim 1, characterised in that diacetylated diamines, organic sulphoxides or a glucocorticoid which is substituted in the 11β position by a hydrophilic group, and mixtures thereof or a mixture consisting of the above-mentioned stimulators and a saturated aliphatic carboxylic acid and the salts thereof are used as stimulator.

3. Process as claimed in claim 1, characterised in that N,N'-diacetylated α,ω-diamines with 4 to 8 carbon atoms, dimethylsulphoxide or glucocorticoids which are substituted in the 11β position by a hydroxy or hydroxymethyl group, and mixtures thereof or a mixture consisting of the above-mentioned stimulators and an alkanoic acid with 3 to 6 carbon atoms and the salts thereof are used as stimulator.

4. Process as claimed in claim 1, characterised in that pentamethylene-bis-acetamide, hexamethylene-bis-acetamide, heptamethylene-bis-acetamide, dimethylsulphoxide, prednisolone, dexamethasone, triamcinolone, hydrocortisone, 11-desoxycortisone and prednisone and mixtures thereof or a mixture consisting of the above-mentioned stimulators and propionic acid, n-butyric acid, isobutyric acid, n-valeric acid, caproic acid and salts thereof with inorganic or organic bases are used as stimulator and the stimulator is added to the cells for a period of 6–72 hours, but preferably 24–48 hours, before the administration of the interferon inducers.

5. Process as claimed in claims 1 to 4, characterised in that the final concentration of stimulator is 0.01 μmol to 300 mmol/l.

6. Process as claimed in claims 1 to 4, characterised in that N,N'-diacetylated diamines with 4 to 8 carbon atoms and/or dimethylsulphoxide are used as stimulator, in a final concentration of 0.1 μmol/l to 250 mmol/l.

**Claims for the Contracting States: IT, LU**

1. Process for the preparation of interferon, characterised in that, before being incubated with viral interferon inducers cells of a permanently cultivatable line of cells of lymphoblastoid origin are treated with stimulators which cause the lymphoblastoid cells which are in a logarithmic growth phase to move out of the growth phase into a resting stage, under optimum growth conditions, this resting stage being defined by the fact that the cells correspond to post-mitotic (G₁ phase) cells with regard to their deoxyribonucleic acid content and no DNA synthesis occurs, and if desired the interferon formed during the incubation is separated and/or purified according to methods known per se.

2. Process as claimed in claim 1, characterised in that a saturated aliphatic carboxylic acid and the salts thereof, diacetylated diamines, organic sulphoxides or a glucocorticoid which is substituted in the 11β position by a hydrophilic group, and mixtures thereof, are used as stimulator.

3. Process as claimed in claim 1, characterised in that an alkanoic acid with 3 to 6 carbon atoms or the salts thereof, N,N'-diacetylated α,ω-diamines with 4 to 8 carbon atoms, dimethylsulphoxide or glucocorticoids which are substituted in the 11β position by a hydroxy or hydroxymethyl group, and mixtures thereof, are used as stimulators.

4. Process as claimed in claim 1, characterised in that propionic acid, n-butyric acid, isobutyric acid, n-valeric acid, caproic acid and the salts thereof with inorganic or organic bases, pentamethylene-bis-acetamide, hexamethylene-bis-acetamide, heptamethylene-bis-acetamide, dimethylsulphoxide, prednisolone, dexamethasone, triamcinolone, hydrocortisone, 11-desoxycortisone and prednisone and mixtures thereof are used as the stimulator and the stimulator is added to the cells for a period of 6–72 hours, but preferably 24–48 hours, before the administration of the interferon inducers.

5. Process as claimed in claims 1 to 4, characterised in that the final concentration of the stimulator is 0.01 μmol to 300 mmol/l.

6. Process as claimed in claims 1 to 4, characterised in that an alkanoic acid with 3 to 5 carbon atoms or the salts thereof, N,N'-diacetylated diamines with 4 to 8 carbon atoms and/or dimethylsulphoxide are used as stimulator, in a final concentration of 0.01 μmol/l to 250 mmol/l.

**Revendications pour les Etats contractants: BE, DE, FR, GB, NL, SE, CH**

1. Procédé pour la préparation d'interféron, caractérisé en ce que des cellules d'une lignée de cellules d'origine lymphoblastique cultivable de façon permanente, avant d'être incubées avec des inducteurs d'interféron viraux, sont traitées par des stimulateurs qui agissent de telle sorte que les cellules lymphoblastiques qui se trouvent en phase de croissance logarithmique, dans des conditions optimales de croissance passent de la phase de croissance à une phase de repos, laquelle est définie en ce que les cellules correspondent, en ce qui concerne leur teneur en acides désoxyribonucléiques, à des cellules post-mitotiques (phase G₁) et qu'aucune synthèse de DNA ne se produit, et qu'éventuellement l'interféron formé lors de l'incubation est séparé et/ou purifié selon des méthodes connues en soi, l'utilisation d'acides carboxyliques aliphatiques saturés rectilignes ou de leurs sels en tant qu'uniques stimulateurs étant exclue.

2. Procédé selon la revendication 1, caractérisé en ce qu'on utilise, en tant que stimulateur, des diamines diacétylées, des sulfoxydes organiques ou un glucocorticoïde qui est substitué en position 11 β par un groupe hydrophile, ainsi que leurs mélanges ou un mélange constitué des stimulateurs mentionnés ci-dessus et d'un acide aliphatique saturé et de ses sels.

3. Procédé selon la revendication 1, caractérisé en ce qu'on utilise, en tant que stimulateur, des α,ω-diamines N, N'-diacétylées avec 4 à 8 atomes de carbone, le dimétyhlsulfoxyde ou des glucocorticoïdes qui sont substitués en position 11 β par un groupe hydroxy ou hydroxyméthyle, ainsi que leurs mélanges ou un mélange constitué des stimulateurs mentionnés ci-dessus et d'un acide alcanoïque avec 3 à 6 atomes de carbone et de ses sels.

4. Procédé selon la revendication 1, caractérisé en ce qu'on utilise, en tant que stimulateur, le pentaméthylènebisacétamide, l'hexaméthylènebisacétamide, l'heptaméthylènebisacétamide, le diméthylsulfoxyde, la prednisolone, la dexaméthasone, la triamcinolone, l'hydrocortisone, la 11-désoxycortisone et la prednisone, ainsi que leurs mélanges ou un mélange constitué des stimulateurs mentionnés ci-dessus et d'acide propionique, d'acide n-butyrique, d'acide isobutyrique, d'acide n-valérique, d'acide caproïque et de leurs sels avec des bases minérales ou organiques et on ajoute le stimulateur aux cellules pour une durée de 6–72 heures, de préférence cependant de 24–78 heures, avant l'addition des inducteurs d'interféron.

5. Procédé selon les revendications 1–4, caractérisé en ce que la concentration finale du stimulateur est de 0,01 μmole–300 mmoles/l.

6. Procédé selon les revendications 1–4, caractérisé en ce qu'on utilise, en tant que stimulateur, des diamines N, N'-diacétylées avec 4 à 8 atomes de carbone et/ou le diméthylsulfoxyde à une concentration finale de 0,1 μmole/l à 250 mmoles/l.

**Revendications pour les Etats contractants: IT, LU**

1. Procédé pour la préparation d'interféron, caractérisé en ce que des cellules d'une lignée de cellules d'origine lymphoblastique cultivable de façon permanente, avant d'être incubées avec des inducteurs d'interféron viraux, sont traitées par des stimulateurs qui agissent de telle sorte que les cellules lymphoblastiques qui se trouvent en phase de croissance logarithmique, dans des conditions optimales de croissance passent de la phase de croissance à une phase de repos, laquelle est définie en ce que les cellules correspondent, en ce qui concerne leur teneur en acides désoxyribonucléiques, à des cellules post-mitotiques (phase $G_1$) et qu'aucune synthèse de DNA ne se produit, et qu'éventuellement l'interféron formé lors de l'incubation est séparé et/ou purifié selon des méthodes connues en soi.

2. Procédé selon la revendication 1, caractérisé en ce qu'on utilise, en tant que stimulateur, un acide carboxylique aliphatique saturé et ses sels, des diamines diacétylées, des sulfoxydes organiques ou un glucocorticoïde qui est substitué en position 11 β par un groupe hydrophile, ainsi que leurs mélanges.

3. Procédé selon la revendication 1, caractérisé en ce qu'on utilise, en tant que stimulateur, un acide alcanoïque avec 3 à 6 atomes de carbone ou ses sels, des α,ω-diamines N,N'-diacétylées avec 4 à 8 atomes de carbone, le diméthylsulfoxyde ou des glucocorticoïdes qui sont substitués en position 11 β par un groupe hydroxy ou hydroxyméthyle, ainsi que leurs mélanges.

4. Procédé selon la revendication 1, caractérisé en ce qu'on utilise, en tant que stimulateur, l'acide propionique, l'acide n-butyrique, l'acide isobutyrique, l'acide n-valérique, l'acide caproïque et leurs sels avec des bases minérales ou organiques, le pentaméthylènebisacétamide, l'hexaméthylènebisacétamide, l'heptaméthylènebisacétamide, le diméthylsulfoxyde, la prednisolone, la dexaméthasone, la triamcinolone, l'hydrocortisone, la 11-désoxycortisone et la prednisone, ainsi que leurs mélanges et on ajoute le stimulateur aux cellules pour une durée de 6–72 heures, de préférence cependant de 24–48 heures, avant l'addition des inducteurs d'interféron.

5. Procédé selon les revendications 1–4, caractérisé en ce que la concentration finale du stimulateur est de 0,01 μmole–300 mmoles/1.

6. Procédé selon les revendications 1–4, caractérisé en ce qu'on utilise, en tant que stimulateur, un acide alcanoïque avec 3 à 5 atomes de carbone ou ses sels, des diamines N, N'-diacétylées avec 4 à 8 atomes de carbone et/ou le diméthylsulfoxyde à une concentration finale de 0,1 μmole/l à 250 mmoles/l.